# EUROPÄISCHE PATENTSCHRIFT

(11) **EP 0 994 693 B1**
(45) Veröffentlichungstag und Bekanntmachung des Hinweises auf die Patenterteilung: **05.12.2001**
(21) Anmeldenummer: 98940191.4
(22) Anmeldetag: 09.07.1998
(51) Int. Cl.: A61K 9/00

(54) **LAGERSTABILE OPHTHALMISCHE ZUSAMMENSETZUNGEN, UMFASSEND DICLOFENAC UND OFLOXACIN**
STORAGE-STABLE OPHTHALMIC COMPOSITIONS COMPRISING DICLOFENAC AND OFLOXACIN
COMPOSITIONS OPHTALMIQUES STABLES AU STOCKAGE CONTENANT DU DICLOFENAC ET DE L'OFLOXACINE

(30) Priorität: 11.07.1997 DE 19729879
(43) Veröffentlichungstag der Anmeldung: 26.04.2000
(73) Patentinhaber: Dr. GERHARD MANN chem.-pharm. Fabrik GmbH, D-13581 Berlin (DE)
(72) Erfinder: BELLMANN, Günther, D-13593 Berlin (DE); CLAUS-HERZ, Gudrun, D-14167 Berlin (DE); KESSLER, Christoph, D-10625 Berlin (DE)
(74) Vertreter: Maiwald Patentanwalts GmbH
(86) Internationale Anmeldenummer: EP9804267
(87) Internationale Veröffentlichungsnummer: WO9902130

(56) Entgegenhaltungen:
- EP-A- 0 242 328
- EP-A- 0 274 714
- EP-A- 0 275 515
- EP-A- 0 306 984
- WO-A-89/06964
- CHEMICAL ABSTRACTS, vol. 122, no. 26, 26. Juni 1995 Columbus, Ohio, US; abstract no. 322527x, VAN WIE BERGAMINI, M. ET AL.: "Ophthalmic formulation based on a steroidal or nonsteroidal antiinflammatory agent and a DNA gyrase-inhibiting antibiotic" Seite 581; XP002900334 & ES 2 065 846 A
- NÜRNBERG, E. ET AL.: "Anforderungen an Augenpräparate" 1991 , SPRINGER-VERLAG , HAGERS HANDBUCH DER PHARMAZEUTISCHEN PRAXIS 5., VOLLSTÄNDIG NEUBEARBEITETE AUFLAGE, BAND 2, SEITEN 639-649 XP002900335 siehe Abschnitt " pH-Wert" , Seite 640; Seite 643, Abschnitte "Hilfsstoffe", "Antioxidanzien", "Tenside" siehe Seite 639 siehe Seite 648, letzter Absatz siehe Seite 649, Absatz 1

## Beschreibung

Die Erfindung betrifft lagerstabile ophthalmische Zusammensetzungen, umfassend Diclofenac bzw. dessen Salze und Ofloxacin in Form seines Racemats, eines seiner Enantiomeren, insbesondere des Levofloxacins, oder des entsprechenden Hydrochlorids. Außerdem betrifft die Erfindung die Verwendung derartiger ophthalmischer Zusammensetzungen zur Behandlung von Entzündungen und/oder Infektionen des Auges sowie zur Infektionsprophylaxe vor und nach Operationen am Auge.

Sowohl die einzelne als auch die kombinierte Verabreichung von Antibiotika und Antiphlogistika zur gleichzeitigen Behandlung von Entzündungen und Infektionen des vorderen Augenabschnitts sind seit langem bekannt. Dabei hat sich insbesondere die Verabreichung von entsprechenden Kombinationspräparaten als vorteilhaft erwiesen.

Bei derartigen Kombinationspräparaten muß sichergestellt sein, daß sich die Inhaltsstoffe hinsichtlich ihrer Wirksamkeit und auch hinsichtlich ihrer Stabilität nicht gegenseitig negativ beeinflussen oder gar chemische Reaktionen miteinander eingehen, die zu unerwünschten Nebenprodukten rühren könnten.

In der PCT-Anmeldung WO90/01933 werden ophthalmische Kombinationspräparate aus Antibiotika und steroidalen Antiphlogistika offenbart, die diesen Kriterien gerecht werden. Als Wirkstoffe werden u.a. Ofloxacin in Kombination mit Dexamethason oder Rimexolon genannt. Wenngleich diese Zusammensetzungen hinsichtlich der Kompatibilität ihrer Inhaltsstoffe und der Lagerstabilität zufriedenstellend sind, sind sie mit den durch die Steroidkomponente bedingten, bekannten nachteiligen Effekten, wie beispielsweise dem Anstieg des intraokularen Druckes, behaftet. Darüber hinaus handelt es sich bei Dexamethason um ein nur schwach-penetrierendes Antiphlogistikum.

Um die durch die Steroidkomponente bedingten nachteiligen Effekte vorbekannter Kombinationspräparate zu überwinden, wurde in der spanischen Patentschrift ES 2065846 vorgeschlagen, anstelle eines steroidalen Antiphlogistikums ein nicht-steroidales Antiphlogistikum mit einem Antibiotikum aus der Gruppe der Gyrase-Inhibitoren zu kombinieren. Exemplifiziert werden ophthalmische Suspensionen und Salben, umfassend die Wirkstoffkombinationen Clobetason/Lomefloxacin, Fluorometholon/Norfloxacin, Dexamethason/Ciprofloxacin sowie Indomethacin/Norfloxacin.

In der europäischen Patentanmeldung EP 0 711 546 werden Kombinationspräprarate für die Ophthalmologie und Otologie offenbart, die das nicht-steroidale Antiphlogistikum Diclofenac bzw. dessen Salze und das Antibiotikum Tobramycin umfassen. Die beschriebenen Formulierungen sind mit erheblichen Stabilitätsproblemen behaftet, die zum einen auf die schlechte Löslichkeit des Diclofenacs und zum anderen auf chemische Wechselwirkungen zwischen den Wirkstoffen zurückgeführt werden. Die beobachteten Stabilitätsprobleme konnten durch sorgfältige Einstellung der Wirkstoffkonzentrationen und des pH-Wertes sowie durch Verwendung geeigneter Lösungsvermittler überwunden werden. Ein Nachteil dieser Zusammensetzung ist darin zu sehen, daß das Tobramycin nicht in das Auge penetrieren kann, also nur zur Behandlung oberflächiger Infektionen geeignet ist.

Aus therapeutischer Sicht ist ein ophthalmisches Kombinationspräparat aus einen penetrierenden Antiphlogistikum und einem penetrierenden Antibiotikum wünschenswert. Als geeignete Wirkstoffe kämen hier vor allem Diclofenac bzw. dessen Salze und Ofloxacin in Form seines Racemats, eines seiner Enantiomeren, insbesondere des Levofloxacins, oder des entsprechenden Hydrochlorids in Betracht.

Bei den ersten Basisuntersuchungen zur vorliegenden Erfindung wurde gefunden, daß bei der Formulierung eines Kombinationsarzneimittels mit den Wirkstoffen Diclofenac und Ofloxacin unerwartete Stabilitätsprobleme auftreten, dergestalt, daß sich ein Zersetzungsprodukt des Ofloxacins, das Ofloxacin-N-oxid, in stärkerem Maße bildet, als es bei Ofloxacin in Monopräparaten der Fall ist. Schon nach einer Lagerzeit von 6 Monaten bildet sich das Ofloxacin-N-oxid in Anwesenheit von Diclofenac zu ca. 2 %, während bei reinen Lösungen von Ofloxacin, also in Abwesenheit von Diclofenac, der Gehalt des Oxids selbst nach längerer Lagerzeit unter 0,2 % liegt.

Aufgabe der vorliegenden Erfindung ist es, ein stabiles Kombinationspräparat, umfassend Diclofenac bzw. dessen Salze und Ofloxacin in Form seines Racemats oder eines seiner Enantiomeren, insbesondere des Levofloxacins, oder des entsprechenden Hydrochlorids bereitzustellen, das zur Behandlung von Entzündungen und/oder Infektionen des Auges sowie zur Infektionsprophylaxe vor und nach Operationen am Auge verwendet werden kann.

Die Lösung dieser Aufgabe wird erfindungsgemäß durch die Merkmale der unabhängigen Ansprüche ermöglicht.

Die abhängigen Ansprüche definieren vorteilhafte Ausgestaltungen der Erfindung.

Es wurde überraschend festgestellt, daß sich die zuvor beschriebene Bildung von Ofloxacin-N-oxid bzw. Levofloxacin-N-oxid durch die Verwendung von üblicherweise in ophthalmischen Präparaten verwendeten Antioxidationsmitteln zurückdrängen läßt.

Um das Diclofenac in Lösung zu halten, ist ein stabiler pH-Wert größer 7 einzuhalten. Üblicherweise erfolgt die Einstellung des pH-Wertes ophthalmischer Präparate mit Puffersystemen.

Im vorliegenden Fall wurde jedoch beobachtet, daß es bei der Pufferung von Diclofenac- und Ofloxacin-haltigen Lösungen auch in Anwesenheit eines Oxidationsmittels während der Lagerzeit zur Bildung von Ausfällungen kam, die vermutlich auf die hohe Salzkonzentration des gepufferten Systems zurückzuführen sind. Diese Ausfällungen konnten auch durch den Zusatz üblicher Lösungsvermittler nicht verhindert werden.

Erfindungsgemäß wurde nun gefunden, daß stabile Kombinationspräparate, umfassend die Wirkstoffe Diclofenac bzw. dessen Salze und Ofloxacin in Form seines Racemats, eines seiner Enantiomeren oder des entsprechenden Hydrochlorids erhalten werden können, indem übliche Antioxidantien zugesetzt werden und auf ein Puffersystem verzichtet wird, d.h. die Einstellung des pH-Wertes allein mit Säure bzw. Alkalien erfolgt.

Die erfindungsgemäßen Formulierungen enthalten 0,001-0,15 Gew.%, vorzugsweise 0,01-0,13 Gew.-%, bevorzugt 0,08-0,12 Gew.-% Diclofenac bzw. dessen Salze und 0,001-0,5 Gew.-%, vorzugsweise 0,01-0,35 Gew. %, bevorzugt 0,1-0,3 Gew.-% Ofloxacin in Form seines Racemats, eines seiner Enantiomeren, insbesondere des Levofloxacins, oder des entsprechenden Hydrochlorids.

Als geeignete Antioxidantien enthalten die erfindungsgemäßen Zusammensetzungen Ascorbinsäure, Citronensäure, Weinsäure, Natriumsulfit oder Natriumdisulfit in einer Menge von 0,001-0,02 Gew.-%, vorzugsweise 0,005-0,015 Gew.-%, bevorzugt 0,008-0,012 Gew.-%. Besonders bevorzugt enthalten die erfindungsgemäßen Zusammensetzungen Natriumdisulfit in einer Konzentration zwischen 0,008 und 0,012 Gew.-%.

Zur Einstellung des pH-Wertes worden bevorzugt HCl und NaOH verwendet. Die erfindungsgemäßen Zusammensetzungen können übliche Lösungsvermittler, wie Polyvidon, etc. in einer Konzentration von 0-5 Gew.-%, vorzugsweise 2-4 Gew.-% und bevorzugt 2,5-3,5 Gew.-% und/oder Polysorbat, etc. in einer Konzentration von 0-2 Gew.-%, vorzugsweise 0,1-1 Gew.-% und bevorzugt 0,3-0,7 Gew.-% enthalten.

Als weitere übliche Zusatzstoffe können die erfindungsgemäßen Zusammensetzungen enthalten:
- Isotonisierungsmittel, wie Natriumchlorid in einer Menge von 0-5 Gew.-%, vorzugsweise 0,3-2 Gew.-%, bevorzugt 0,5-1,25 Gew.-% oder Sorbitol in einer Menge von 0-10 Gew.-%, vorzugsweise 2-6 Gew.-%, bevorzugt 3-5 Gew.-%;
- chelatisierende Mittel, wie EDTA oder dessen Natriumsalz in einer Menge von 0-0,002 Gew.-%, bevorzugt 0,0008-0,0012 Gew.-%;
- Konservierungsmittel, wie Benzalkoniumchlorid in einer Menge von 0-1,0 Gew.-%, vorzugsweise 0,01-0,5 Gew.-%, bevorzugt 0,02-0,04 Gew.-%.

Die erfindungsgemäßen Kombinationspräparate können als Augentropfen, Suspensionen, Salben oder Gele formuliert werden. Besonders bevorzugt ist die Formulierung als Augentropfen.

Die folgenden Ausführungsbeispiele dienen nur der Erläuterung der Erfindung und stellen keinerlei Einschränkung dar.

### BEISPIEL 1

Ansatzgröße: 20 l

| Bestandteile | Menge |
|---|---|
| Diclofenac-Natrium | 0,020 kg |
| Ofloxacin | 0,060 kg |
| Kollidon 25 | 0,600 kg |
| Natriumdisulfit | 0,002 kg |
| Natriumchlorid | 0,150 kg |
| 1 N HCl zur pH-Werteinstellung | |
| 1 N NaOH zur pH-Werteinstellung | |
| Wasser | 19,404 kg |

### BEISPIEL 2

Ansatzgröße: 20 l

| Bestandteile | Menge |
|---|---|
| Diclofenac-Natrium | 0,020 kg |
| Levofloxacin | 0,060 kg |
| Natriumedetat | 0,002 kg |
| Kollidon 25 | 0,600 kg |
| Natriumdisulfit | 0,002 kg |
| Natriumchlorid | 0,150 kg |
| Polysorbat | 0,100 kg |
| 1 N HCl zur pH-Werteinstellung | |
| 1 N NaOH zur pH-Werteinstellung | |
| Wasser | 19,304 kg |

### BEISPIEL 3

Ansatzgröße: 20 l

| Bestandteile | Menge |
|---|---|
| Diclofenac-Natrium | 0,020 kg |
| Ofloxacin | 0,060 kg |
| Kollidon 25 | 0,600 kg |
| Natriumdisulfit | 0,002 kg |
| Sorbitol | 0,800 kg |
| Polysorbat | 0,100 kg |
| 1 N HCl zur pH-Werteinstellung | |
| 1 N NaOH zur pH-Werteinstellung | |
| Wasser | 18,838 kg |

### BEISPIEL 4

Ansatzgröße: 20 l

| Bestandteile | Menge |
|---|---|
| Diclofenac-Natrium | 0,020 kg |
| Ofloxacin | 0,060 kg |
| Kollidon 25 | 0,600 kg |
| Natriumdisulfit | 0,002 kg |
| Sorbitol | 0,800 kg |
| Benzalkoniumchlorid | 0,006 kg |
| Polysorbat | 0,100 kg |
| 1 N HCl zur pH-Werteinstellung | |
| 1 N NaOH zur pH-Werteinstellung | |
| Wasser | 18,832 kg |

Die exemplifizierten Zusammensetzungen waren über lange Zeit stabil und wiesen nur niedrige Gehalte Ofloxacin-N-oxid auf, die im Laufe der Lagerzeit nicht zunahmen.

Die erfindungsgemäßen stabilen Zusammensetzungen können zur Behandlung von Infektionen und/oder Entzündungen des Auges sowie zur Infektionsprophylaxe vor und nach Operationen am Auge verwendet werden.

In der folgenden Tabelle I werden die Ergebnisse verschiedener Versuchsreihen gegenübergestellt.

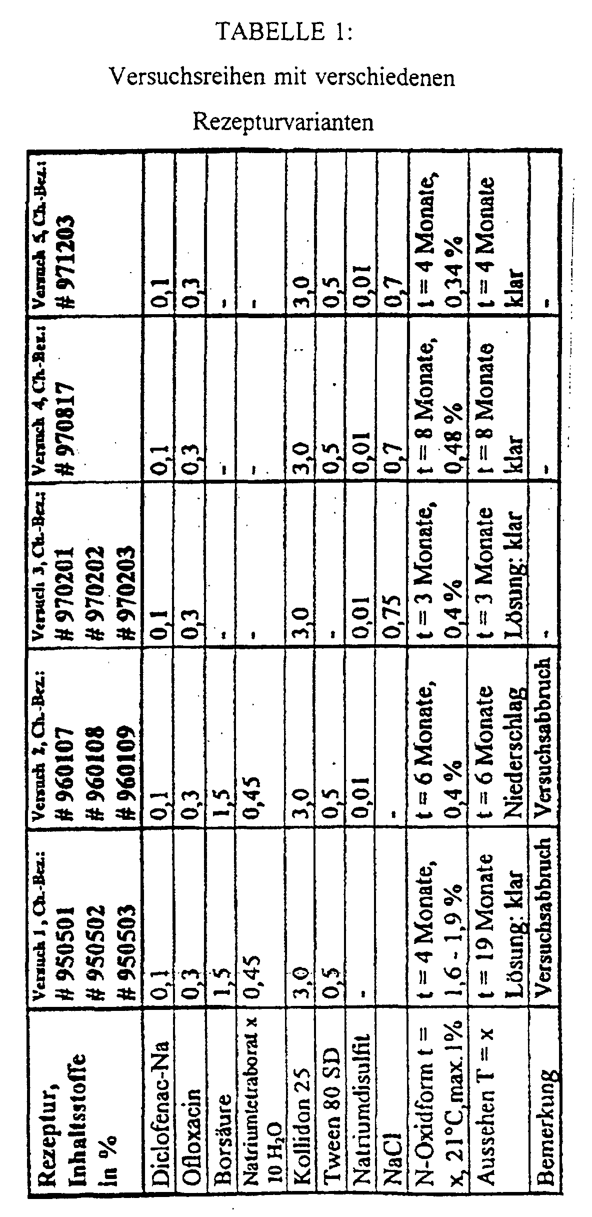

In der Versuchsreihe 1 wurde eine Zusammensetzung untersucht, die neben den Wirkstoffen Diclofenac-Na und Ofloxacin das Puffersystem Borsäure/Natriumtetraborat sowie die Hilfsstoffe Kollidon 25 und Tween 80 SD enthält. Zwar wurde eine klare Lösung erhalten, allerdings wurden bereits nach 4 Monaten 1,6 - 1,9% des Ofloxacin-N-oxids gebildet.

Wie aus der Versuchsreihe 2 hervorgeht, konnte die Bildung des Ofloxacin-N-oxids durch Zusatz von Natriumdisulfit deutlich zurückgedrängt werden, allerdings kam es bei den in dieser Versuchsreihe untersuchten Zusammensetzungen zur Bildung eines Niederschlags.

Wie die Versuchsreihen 3, 4 und 5 zeigen, können erfindungsgemäß klare Lösungen mit geringen Gehalten an Ofloxacin-N-oxid erhalten werden, wenn man Natriumdisulfit zusetzt und gleichzeitig auf das Puffersystem verzichtet.

## Patentansprüche

1. Stabile ophthalmische Zusammensetzung für die topische Verabreichung,
**dadurch gekennzeichnet, daß** sie einen pH-Wert größer ca. 7 aufweist und Diclofenac bzw. eines seiner Salze, Ofloxacin in Form seines Racemats, eines seiner Enantiomere, insbesondere des Levofloxacins, oder des entsprechenden Hydrochlorids sowie mindestens ein Antioxidationsmittel umfaßt.

2. Ophthalmische Zusammensetzung gemäß Anspruch 1,
**dadurch gekennzeichnet, daß** der pH-Wert zwischen ca. 7 und ca. 7,4 liegt.

3. Ophthalmische Zusammensetzung nach Anspruch 1 oder 2,
**dadurch gekennzeichnet, daß** das Antioxidationsmittel aus der Gruppe, umfassend Ascorbinsäure, Citronensäure, Weinsäure, Natriumsulfit und Natriumdisulfit ausgewählt ist.

4. Ophthalmische Zusammensetzung gemäß Anspruch 3,
**dadurch gekennzeichnet, daß** das Antioxidationsmittel Natriumdisulfit ist.

5. Ophthalmische Zusammensetzung nach einem der Ansprüche 1 bis 4,
**dadurch gekennzeichnet, daß** sie weiterhin ein Isotonisierungsmittel und/oder einen Lösungsvermittler und/oder ein chelatisierendes Mittel und/oder ein Konservierungsmittel umfaßt.

6. Ophthalmische Zusammensetzung gemäß Anspruch 5, wobei der Lösungsvermittler Polyvidon und/oder Polysorbat ist.

7. Ophthalmische Zusammensetzung gemäß Anspruch 5 oder Anspruch 6,
**dadurch gekennzeichnet, daß** das chelatisierende Mittel EDTA oder dessen Natriumsalz ist.

8. Ophthalmische Zusammensetzung nach einem der Ansprüche 1 bis 7,
**dadurch gekennzeichnet, daß** sie 0,001-0,15 Gew.-%, vorzugsweise 0,001-0,13 Gew.-%, bevorzugt 0,08-0,12 Gew.-% Diclofenac oder dessen Natriumsalz,
0,01-0,5 Gew.-%, vorzugsweise 0,01-0,35 Gew.-%, bevorzugt 0,1-0,3 Gew.-% Ofloxacin, oder eines seiner Enantiomeren, insbesondere Levofloxacin, oder das entsprechende Hydrochlorid,
0,001-0,02 Gew.-%, vorzugsweise 0,005-0,015 Gew.-%, bevorzugt 0,008-0,012 Gew.-% Natriumdisulfit,
0-5 Gew.-%, vorzugsweise 2-4 Gew.-%, bevorzugt 2,5-3,5 Gew.-% Polyvidon und/oder Polysorbat in einer Konzentration von
0-2 Gew.-%, vorzugsweise 0,1-1 Gew.-% und bevorzugt 0,3-0,7 Gew.-%,
0-5 Gew.-%, vorzugsweise 0,3-2 Gew.-%, bevorzugt 0,5-1,25 Gew.-% Natriumchlorid, oder 0-10 Gew.-%, vorzugsweise 2-6 Gew.-%, bevorzugt 3-5 Gew.-% Sorbitol,
0-1,0 Gew.-%, vorzugsweise 0,01-0,5 Gew.-%, bevorzugt 0,02-0,04 Gew.-% Benzalkonuiumchlorid,
0-0,002 Gew.-%, bevorzugt 0,0008-0,0012 Gew.-% Natriumedetat, sowie Alkalihydroxid bzw. -säure zur Einstellung des pH-Wertes und im übrigen Wasser umfaßt.

9. Verwendung der Zusammensetzung gemäß einem der Ansprüche 1 bis 8 zur Herstellung eines Arzneimittels für die Behandlung von Entzündungen und/oder Infektionen des Auges oder die Infektionsprophylaxe vor und nach Operationen am Auge.

## Claims

1. Stable ophthalmic composition for topical administration,
**characterised in that** it has a pH value greater than approx. 7 and comprises diclofenac or one of its salts, ofloxacin in the form of its racemate, of one of its enantiomers, in particular levofloxacin, or of the corresponding hydrochloride, and as least one antioxidant.

2. Ophthalmic composition according to claim 1,
**characterised in that** the pH value lies between approx. 7 and approx. 7.4.

3. Ophthalmic composition according to claim 1 or 2,
**characterised in that** the antioxidant is selected from the group comprising ascorbic acid, citric acid, tartaric acid, sodium sulfite and sodium disulfite.

4. Ophthalmic composition according to claim 3,
**characterised in that** the antioxidant is sodium disulfite.

5. Ophthalmic composition according to any one of claims 1 to 4,
**characterised in that** it comprises in addition an isotonizing agent and/or a solubilizer and/or a chelating agent and/or a preservative.

6. Ophthalmic composition according to claim 5,
in which the solubilizer is polyvidone and/or polysorbate.

7. Ophthalmic composition according to claim 5 or claim 6,
**characterised in that** the chelating agent is EDTA or its sodium salt.

8. Ophthalmic composition according to any one of claims 1 to 7,
**characterised in that** comprises 0.001 - 0.15 wt %, for preference 0.001 - 0.13 wt %, preferably 0.08 - 0.12 wt % of diclofenac or its sodium salt,
0.01 - 0.5 wt %, for preference 0.01 - 0.35 wt %, preferably 0.1 - 0.3 wt % of ofloxacin, or of one of its enantiomers, in particular levofloxacin, or the corresponding hydrochloride,
0.001 - 0.02 wt %, for preference 0.005 - 0.015 wt %, preferably 0.008 - 0.012 wt % of sodium disulfite,
0 - 5 wt %, for preference 2 - 4 wt %, preferably 2.5 - 3.5 wt % of polyvidone and/or polysorbate in a concentration of 0 - 2 wt %, for preference 0.1 - 1 wt % and preferably 0.3 - 0.7 wt %,
0 - 5 wt %, for preference 0.3 - 2 wt %, preferably 0.5 - 1.25 wt % of sodium chloride, or 0-10 wt %, for preference 2 - 6 wt %, preferably 3 - 5 wt % of sorbitol,
0 - 1.0 wt %, for preference 0.01 - 0.5 wt %, preferably 0.02 - 0.04 wt % of benzalkonium chloride,
0 - 0.002 wt %, preferably 0.0008 - 0.0012 wt % of sodium edetate, as well as alkali hydroxide or acid for adjusting the pH value and for the rest water.

9. Use of the composition according to any one of claims 1 to 8 for preparing a drug for the treatment of inflammations and/or infections of the eye or for prophylaxis before and after operations on the eye.

## Revendications

1. Composition ophtalmique stable pour l'administration locale, **caractérisée en ce qu'**elle présente une valeur de pH supérieur à environ 7 et comprend du diclofénac respectivement un de ses sels, de l'ofloxacine sous forme de son racémate, d'un de ses énantiomères en particulier de la lévofloxacine, ou du chlorhydrate correspondant, ainsi qu'au moins un antioxydant.

2. Composition ophtalmique selon la revendication 1, **caractérisée en ce que** la valeur de pH se situe entre environ 7 et environ 7,4.

3. Composition ophtalmique selon la revendication 1 ou 2, **caractérisée en ce que** l'antioxydant est choisi parmi le groupe comprenant l'acide ascorbique, l'acide citrique, l'acide tartrique, le sulfite de sodium et le disulfite de sodium.

4. Composition ophtalmique selon la revendication 3, **caractérisée en ce que** l'antioxydant est le disulfite de sodium.

5. Composition ophtalmique selon l'une quelconque des revendications 1 à 4, **caractérisée en ce qu'**elle comprend en outre un agent d'isotonisation et/ou un dissolvant et/ou un agent de chélation et/ou un conservant.

6. Composition ophtalmique selon la revendication 5, **caractérisé en ce que** le dissolvant est de la polyvidone et/ou un polysorbat.

7. Composition ophtalmique selon la revendication 5 ou 6, **caractérisé en ce que** l'agent de chélation est le EDTA ou son sel de sodium.

8. Composition ophtalmique selon l'une quelconque des revendications 1 à 7, **caractérisée en ce qu'**elle comprend du diclofénac ou son sel de sodium à concurrence de 0,001 à 0,15 % en poids de préférence de 0,001 à 0,13 % en poids, de manière avantageuse de 0,08 à 0,12 % en poids,
de l'ofloxacine ou un de ses énantiomères en particulier la lévofloxacine, ou le chlorhydrate correspondant à concurrence de 0,01 à 0,5 % en poids, de préférence de 0,01 à 0,35 % en poids, de manière avantageuse de 0,1 à 0,3 % en poids,
du disulfite de sodium à concurrence de 0,01 à 0,02 % en poids, de préférence de 0,005 à 0,015 % en poids, de manière avantageuse de 0,008 à 0,012 % en poids,
de la polyvidone à concurrence de 0 à 5 % en poids, de préférence de 2 à 4 % en poids, de manière avantageuse de 2,5 à 3,5 % en poids et/ou du polysorbat en une concentration de 0 à 2 % en poids, de préférence de 0,1 à 1 % en poids, de manière avantageuse de 0,3 à 0,7 % en poids
du chlorure de sodium à concurrence de 0 à 5 % en poids, préférence de 0,3 à 2 % en poids, de manière avantageuse de 0,5 à 1,25 % en poids, ou du sorbitol à concurrence de 0 à 10 % en poids, de préférence de 2 à 6 % en poids, de manière avantageuse de 3 à 5 % en poids,
du chlorure de benzalkonium à concurrence de 0 à 1,0 % en poids, de préférence de 0,01 à 0,5 % en poids, de manière avantageuse de 0,02 à 0,04 % en poids,
de l'édétate de sodium, ainsi qu'un hydroxyde de métal alcalin, respectivement un acide pour le réglage de la valeur du pH à concurrence de 0 à 0,002 % en poids, de préférence de 0,0008 à 0,0012 % en poids, de manière avantageuse de 0,02 à 0,04 % en poids et, pour le reste, de l'eau.

9. Utilisation de la composition selon l'une quelconque des revendications 1 à 8 pour la préparation d'un médicament pour le traitement d'inflammations et/ou d'infections de l'oeil ou pour la prophylaxie de l'infection avant et après des opérations sur l'oeil.
